(19) Europäisches Patentamt
European Patent Office
Office européen des brevets

(11) EP 3 527 536 A1

(12) **EUROPEAN PATENT APPLICATION**

(43) Date of publication:
**21.08.2019 Bulletin 2019/34**

(51) Int Cl.:
*C01F 7/00* (2006.01)  *C07C 41/09* (2006.01)
*C07C 43/13* (2006.01)  *B01J 23/00* (2006.01)

(21) Application number: **18156962.5**

(22) Date of filing: **15.02.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: SCG Chemicals Co., Ltd.
**Bangkok 10800 (TH)**

(72) Inventors:
• **Wangriya, Aunchana**
 **10800 Bangkok (TH)**
• **Ngamcharussrivichai, Chawalit**
 **11110 Nonthaburi (TH)**
• **Sangkhum, Prissana**
 **80000 Nakhon Si Thammarat (TH)**

(74) Representative: **Scholz, Volker**
 **Boehmert & Boehmert**
 **Anwaltspartnerschaft mbB**
 **Pettenkoferstrasse 22**
 **80336 München (DE)**

(54) **METHOD FOR PREPARING A CAMGAL MIXED OXIDE, A CAMGAL MIXED OXIDE OBTAINABLE THIS WAY AND THE USE THEREOF FOR OLIGOMERIZATION OF GLYCEROL**

(57) The present invention relates to a method for preparing a CaMgAl mixed oxide comprising the steps: a) providing a modified layered double hydroxide of the formula (I)

$$[Mg_{1-x}Al_x(OH)_2]CO_3 \cdot b(H_2O) \cdot c(AMO\text{-solvent}) \qquad (I)$$

wherein in formula (I) 0<x<0.9; b is from o to 10, preferably 1 to 10; c is from o to 10, preferably 1 to 10, and the AMO-solvent is an organic solvent miscible with water; b) calcining the modified layered double hydroxide; c) reacting the calcined modified layered double hydroxide with a calcium salt in the presence of an organic acid; and d) calcining the product obtained in step c) to obtain the CaMgAl mixed oxide; a CaMgAl mixed oxide obtainable this way; and the use thereof.

Fig. 1

EP 3 527 536 A1

**Description**

The presented invention relates to a CaMgAl mixed oxide, the preparation thereof and a method for the production of polyglycerols using the same as a catalyst.

A variety of mixed oxides comprising Ca, Mg or Al or mixtures thereof are in the prior art. Likewise, the use of respective mixed oxides for catalyzing the production of oligo- and polyglycerols is described.

WO2010/044531 A1 discloses the production of linear polyglycerols via etherification of glycerol using $(CaO)_a$* $(Ca_{12}Al_{14}O_{33})_{100-a}$, wherein "a" refers to a weight ratio of CaO based on 100 parts by weight of the total catalyst. The respective metal oxide catalysts were prepared from CaAl layered double hydroxides (LDHs) via co-precipitation followed by calcination. However, the method suffers from a variety of disadvantages, for example difficulties to control the metal composition and low diglycerols selectivity.

US 5,721,305 A discloses the polymerization of glycerol in the presence of anionic clay catalysts. In this regard, the use of MgAl hydrotalcite, prepared by co-precipitation, is mentioned to be preferred. However, this method suffers from the disadvantages of high reaction temperature, low diglycerols selectivity and low glycerol conversion rates.

Pérez-Barrado et al., Chemical Engineering Journal, 2015, 264, 547 discloses the etherification of glycerol catalyzed by MgAl LDH and CaAl LDH. According to this disclosure, the LDHs were prepared by co-precipitation followed by calcination at 450 °C. However, the method suffers from low diglycerols selectivity and high acrolein selectivity.

Garcia-Sancho et al., Catalysis Today, 2011, 167, 84 is related to the use of MgAl LDH-derived mixed oxides, which are obtainable by co-precipitation or urea hydrolysis and the use thereof as base catalysts for glycerol etherification. However, this method suffers from low glycerol conversion.

Gholami et al., Journal of the Taiwan Institute of Chemical Engineers, 2013, 44, 117 discloses glycerol etherification catalyzed by $Ca_{1+x}Al_{1-x}La_xO_3$ composites, which were synthesized via co-precipitation followed by calcination at 560 °C. However, this method has several disadvantages, in particular requires high reaction temperature and shows low diglycerols selectivity.

It is, therefore, the object of the present invention to provide mixed oxide materials (or methods for preparing the same) overcoming drawbacks of the prior art, in particular mixed oxide materials, which may be used as catalysts in the glycerol oligomerization for improving diglycerol selectivity.

This object is first of all achieved by a method for preparing a CaMgAl mixed oxide comprising the steps: a) providing a modified layered double hydroxide of the formula (I)

$$[Mg_{1-x}Al_x(OH)_2]CO_3 \cdot b(H_2O) \cdot c(AMO\text{-}solvent) \qquad (I)$$

wherein in formula (I) 0<x<0.9; b is from 0 to 10, preferably 1 to 10; c is from 0 to 10, preferably 1 to 10, and the AMO-solvent is an organic solvent miscible with water; b) calcining the modified layered double hydroxide; c) reacting the calcined modified layered double hydroxide with a calcium salt in the presence of an organic acid; and d) calcining the product obtained in step c) to obtain the CaMgAl mixed oxide.

In this regard, it may be preferred that in the compound of formula (I) the ratio of Mg:Al is from 2 to 4.

The modified layered double hydroxide of the above formula (I) may be prepared by a method comprising precipitation of a layered double hydroxide and dispersing the same in the AMO-solvent (followed by a drying step). Further details as to the method of preparing the modified layered double hydroxide provided in the above step a) can be taken from the published patent applications WO 2014/051530 or WO 2015/144778.

In a preferred embodiment, the AMO-solvent is a solvent having a polarity (P) in the range from 3.8 to 9. Solvent polarity (P) is defined based on experimental solubility data reported by Snyder and Kirkland (Introduction to modern liquid Chromatography, 2nd Edition; John Wiley and Sons, New York, 1979; pp 248-250). Particularly preferred solvents in this regard are acetone, acetonitrile, dimethylformamide, dimethyl sulphoxide, dioxane, ethanol, methanol, n-propanol, isopropanol or tetrathydrofuran. The most preferred solvents are methanol and acetone. Best results were achieved using acetone.

It is further preferred that the amount of calcium in the CaMgAl mixed oxide is 2-10 wt.%, preferably 5 to 8 wt.%, with respect to the weight of the calcined modified layered double hydroxide. By using the preferred amounts of calcium in the CaMgAl mixed oxide, best results as to diglycerols selectivity and diglycerols yield were achieved.

Furthermore, it is preferred that calcining in step b) is from 300 to 700 °C, preferably for 1 to 10h.

It is further preferred that the organic acid used in step c) is selected from citric acid, malic acid, tartaric acid, oxalic acid, succinic acid, formic acid, acetic acid.

Furthermore, it is preferred that reacting in step c) is an aqueous solution. Alternatively, other solvents, such as short chain alcohols, such as methanol and ethanol, suitable to dissolve the calcium salt may be used instead of water as a solvent in step c).

It is further preferred that reacting of step c) is in the further presence of a weak base, preferably ammonium hydroxide.

Ammonium hydroxide is ammonia water (herein 30 wt.% $NH_3$ in water). It is also possible to use organic amines, such as methylamine, ethylamine, to adjust the pH of citric acid solution. Methylamine or ethylamine in water, methanol and ethanol, at various concentrations, are available commercially. Alkaline hydroxides (strong bases), such as NaOH and KOH, are cheaper, and can be used for the pH adjustment, but the metal ions remain in the final mixed oxides and affect the catalysts basicity.

In a further embodiment reacting in step c) is at a pH from 5 to 7.

Furthermore, it is preferred that a step of drying the material obtained in step c) is carried out before performing step d)

It is further preferred that calcining in step d) is at a temperature from 450 to 900 °C.

The object is further achieved by a CaMgAl mixed oxide obtainable by the inventive method.

Furthermore, the object is achieved by the use of the inventive CaMgAl mixed oxide in the oligomerization of glycerol.

Preferably, the oligomerization includes preparing diglycerols from glycerol.

Finally, the object is achieved by a method for oligomerization of glycerol comprising a step of contacting glycerol and the inventive CaMgAl mixed oxide.

In this regard, it is preferred that the amount of CaMgAl mixed oxide is from 2 to 10 wt.-%, preferably 2 to 5 wt.%, preferably 2.5 to 4.5 wt.%, even more preferred 2.5 to 3.5 wt.%, with respect to the weight of glycerol. In this way, best diglycerols selectivity was obtained.

Furthermore, it is preferred that contacting is carried out at a temperature from 150 to 300 °C, preferably 200 to 230 °C. In the above temperature ranges, best diglycerols selectivity and/or diglycerol conversion level was achieved.

It is therefore preferred that the contacting is for 1 to 48 h, preferably 4 to 40 h, even more preferred 8 to 32 h. In the preferred time ranges, best diglycerols selectivity and diglycerols yield were achieved.

It was surprisingly found by the inventors that the inventive method, in particular the key step c) thereof of reacting an AMO-$Mg_3$Al-$CO_3$ LDH material with a calcium salt in the presence of an organic acid results in a CaMgAl mixed oxide material having unique structural properties and, therefore, showing improved catalytic activity in the oligomerization of glycerol in particular is suitable to improve the diglycerols selectivity during oligomerization of glycerol.

In the following, the invention will be described in greater detail referring to the specific examples and Figures, without, however, the intention to respectively limit the scope of the invention.

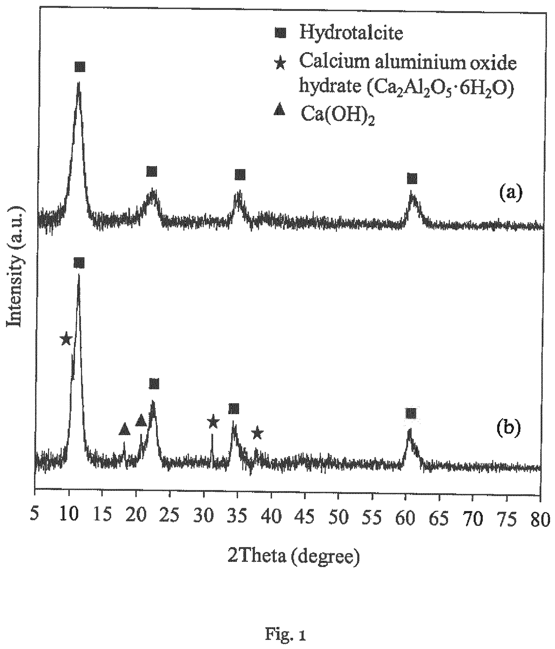

**Figure 1** XRD patterns of reconstructed 7.5%Ca-MgAl LDH (a) prepared by method according to the invention and (b) prepared by conventional impregnation.

## Examples

### Preparation of CaMgAl mixed oxide

### Comparative example A

Modified layered double hydroxide of the formula (I) as described above (AMO-LDH) was prepared according to WO2014051530. A metal precursor solution was prepared by dissolving 1.575 mol (403.85 g) of $Mg(NO_3)_2 \cdot 6H_2O$ and 0.525 mol (196.94 g) of $Al(NO_3)_3 \cdot 9H_2O$ in 700 mL deionized water. The metal precursor was added dropwise into a 700 mL of $Na_2CO_3$ (0.315 mol, 33.39 g) solution. The pH of precipitation solution was adjusted to 10 using a NaOH solution in deionized water (4 M). The precipitant was aged at room temperature for about 3 h. LDH was collected by filtration, washing with deionized water until the filtrate pH was neutral, and rinsing with ethanol. The washed "wet cake" was dispersed in ethanol. After stirring for about 1 h, the sample was filtered. The final AMO-LDH product was dried overnight in the fume hood at room temperature and then in an oven at 110 °C for 12 h.

### Example 1

AMO-LDH, prepared in the same manner as Comparative example A, was calcined, to obtain mixed oxide of Mg and Al, in a muffle furnace at 450-550 °C for 1-5 h. Citric acid solution was prepared by dissolving 2.67 g of citric acid in 2.67 g of deionized water, and adding ammonium hydroxide (30 wt.% ammonia in water) to adjust the solution pH to 5-7. Then, 8.84 g of $Ca(NO_3)_2 \cdot 4H_2O$ were dissolved in the citric acid solution, followed by slowly dropping the Ca solution onto 20 g of the calcined AMO-LDH under stirring. The wet powder was dried in an oven at 100 °C overnight to obtain reconstructed CaMgAl LDH. The sample was analyzed using X-ray diffraction method. Then, it was calcined in a muffle furnace at 650-800 °C for 5 h. The CaMgAl mixed oxide (CaMgAl LDO) with 7.5 wt.% of Ca content was obtained.

Figure 1(a) shows XRD pattern of reconstructed CaMgAl LDH with 7.5 wt.% of Ca content. It consists of pure hydrotalcite phase. There was no separated Ca phase and impurities.

**Example 2**

Example 2 was prepared in the same manner as Example 1, except 5.89 g of $Ca(NO_3)_{2.4}H_2O$ was used to obtain 5 wt.% Ca content.

**Conventional impregnation (Comparative Example B)**

AMO-LDH was prepared and calcined following the method of the invention above. Then, it was impregnated with $Ca(NO_3)_2 \cdot 4H_2O$ in water by slowly dropping 8.84 g of $Ca(NO_3)_2 \cdot 4H_2O$ in water on the calcined AMO-LDH under stirring. The wet powder was dried in an oven at 100 °C overnight to obtain the reconstructed CaMgAl LDH. Then, it was calcined in a muffle furnace at 650-800 °C for 5 h. The CaMgAl mixed oxide with 7.5 wt% of Ca content was obtained.
Figure 1(b) shows the reconstructed CaMgAl LDH prepared by conventional impregnation. It consists of $Ca(OH)2$ and $CaCO_3$ as impurity phases.

**Production of short-chain polyglycerols**

50 g of glycerol were added into a three-necked round-bottom flask equipped with a $N_2$ gas line and Dean-Stark apparatus to continuously remove water generated as a by-product. The glycerol was heated to the reaction temperature under stirring using a heating mantle. Then, the CaMgAl catalyst was added into the flask. The reaction was carried out with 3 wt.% of catalyst loading level with respect to the weight of glycerol used at temperature of 220 °C for 24 h.
The reaction product was diluted with 100 mL of methanol to reduce product viscosity, and then filtered to separate the catalyst. The methanol was removed by rotary evaporator. The product was analyzed for the glycerol conversion and the products yield using a gas chromatography. The glycerol conversion, diglycerols yield and diglycerols selectivity were calculated by the following equations.

$$Glycerol\ conversion\ (mol\%) = \frac{Mole\ of\ converted\ glycerol}{Mole\ of\ initial\ glycerol} \times 100$$

$$Diglycerols\ selectivity\ (\%) = \frac{Mole\ of\ diglycerols}{Mole\ of\ converted\ glycerol} \times 100$$

Comparative examples A shows 36% glycerol conversion and 51% diglycerols selectivity, while comparative example B shows 55% glycerol conversion and 50% diglycerols selectivity. The inventive examples 1 and 2 show higher diglycerols selectivity of 78% and 66%, respectively because it consists pure phase of CaMgAl LDO resulting in high dispersion of Ca atoms. The interaction between Ca, Mg and Al oxides formed a synergetic mixed oxide complex, which provided an enhanced basicity and contributed to the high catalytic performance during glycerol etherification. While, the conventional CaMgAl LDO consists of less basic oxides, and gives low diglycerols selectivity.
The features disclosed in the foregoing description and in the claims may, both separately and in any combination be material for realizing the invention in diverse forms thereof.

**Claims**

1. Method for preparing a CaMgAl mixed oxide comprising the steps:

   a) providing a modified layered double hydroxide of the formula (I)

   $$[Mg_{1-x}Al_x(OH)_2]CO_3 \cdot b(H_2O) \cdot c(AMO\text{-}solvent) \qquad (I)$$

   wherein in formula (I) 0<x<0.9; b is from 0 to 10, preferably 1 to 10; c is from 0 to 10, preferably 1 to 10, and the AMO-solvent is an organic solvent miscible with water;
   b) calcining the modified layered double hydroxide;
   c) reacting the calcined modified layered double hydroxide with a calcium salt in the presence of an organic acid; and
   d) calcining the product obtained in step c) to obtain the CaMgAl mixed oxide.

2. Method according to claim 1, wherein the amount of the calcium salt is 5-10 wt.%, preferably 7 to 8 wt.%, with respect to the weight of the calcined modified layered double hydroxide.

3. Method according to claim 1 or 2, wherein calcining in step b) is from 300 to 700°C, preferably for 1 to 10 h.

4. Method according to any of the preceding claims, wherein reacting of step c) is in the further presence of a weak base, preferably ammonium hydroxide.

5. Method according to any of the preceding claims, wherein reacting in step c) is at a pH from 5 to 7.

6. Method according to any of the preceding claims further comprising a step of drying the material obtained in step c) before performing step d).

7. Method according to any of the preceding claims, wherein calcining in step d) is at a temperature from 450 to 900 °C.

8. Method according to any of the preceding claims, wherein the organic acid is selected from citric acid, malic acid, tartaric acid, oxalic acid, succinic acid, formic acid, acetic acid.

9. Method according to any of the preceding claims, wherein reacting in step c) is an aqueous solution.

10. CaMgAl mixed oxide obtainable by limited according to any of the preceding claims.

11. Use of the CaMgAl mixed oxide according to claim 10 in the oligomerization of glycerol.

12. Use according to claim 11, wherein the oligomerization includes preparing diglycerols from glycerol.

13. Method for oligomerization of glycerol comprising a step of contacting glycerol and the CaMgAl mixed oxide according to claim 10.

14. Method according to claim 13, wherein the amount of CaMgAl mixed oxide is from 2 to 10 wt.% with respect to the weight of glycerol.

15. Method according to claim 13 or 14, wherein contacting is carried out at a temperature from 150 to 300 °C, preferably 200 to 230 °C.

Fig. 1

Europäisches
Patentamt

European
Patent Office

Office européen
des brevets

# EUROPEAN SEARCH REPORT

Application Number

EP 18 15 6962

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YIDONG ZOU ET AL: "Controllable Synthesis of Ca-Mg-Al Layered Double Hydroxides and Calcined Layered Double Oxides for the Efficient Removal of U(VI) from Wastewater Solutions", ACS SUSTAINABLE CHEMISTRY & ENGINEERING, vol. 5, no. 1, 3 January 2017 (2017-01-03), pages 1173-1185, XP055492406, US ISSN: 2168-0485, DOI: 10.1021/acssuschemeng.6b02550 * figure 5b * * p. 1177, final paragraph * | 10 | INV. C01F7/00 C07C41/09 C07C43/13 B01J23/00 |
| X | & YIDONG ZOU ET AL: "Supporting Informtion for Controllable Synthesis of Ca-Mg-Al Layered Double Hydroxides and Calcined Layered Double Oxides for the Efficient Removal of U(VI) from Wastewater Solutions", ACS SUSTAINABLE CHEMISTRY & ENGINEERING, vol. 5, no. 1, 3 January 2017 (2017-01-03), pages 1173-1185, XP055492162, US ISSN: 2168-0485, DOI: 10.1021/acssuschemeng.6b02550 * Synthesis (p. S2) * * table S1 * | 10 | |
| A,D | WO 2014/051530 A2 (SCG CHEMICALS CO LTD [TH]; O'HARE DERMONT [GB]; WANG QIANG [GB]) 3 April 2014 (2014-04-03) * the whole document * | 1-9 | TECHNICAL FIELDS SEARCHED (IPC) C01F C07C B01J |
| A,D | WO 2010/044531 A2 (KOREA RES INST CHEM TECH [KR]; KCI LTD [KR]; HAN YOO HAN [KR]; KIM HYU) 22 April 2010 (2010-04-22) * the whole document * | 11-15 | |

The present search report has been drawn up for all claims

| Place of search | Date of completion of the search | Examiner |
|---|---|---|
| Munich | 16 July 2018 | Stratford, Katja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 15 6962

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2018

| Patent document cited in search report | | Publication date | Patent family member(s) | | Publication date |
|---|---|---|---|---|---|
| WO 2014051530 | A2 | 03-04-2014 | CN | 104703918 A | 10-06-2015 |
| | | | EP | 2900601 A2 | 05-08-2015 |
| | | | JP | 2015535797 A | 17-12-2015 |
| | | | KR | 20150063380 A | 09-06-2015 |
| | | | SG | 11201500993U A | 30-03-2015 |
| | | | TW | 201434740 A | 16-09-2014 |
| | | | US | 2015238927 A1 | 27-08-2015 |
| | | | WO | 2014051530 A2 | 03-04-2014 |
| WO 2010044531 | A2 | 22-04-2010 | KR | 20100041000 A | 22-04-2010 |
| | | | WO | 2010044531 A2 | 22-04-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010044531 A1
- US 5721305 A
- WO 2014051530 A
- WO 2015144778 A

**Non-patent literature cited in the description**

- **PÉREZ-BARRADO et al.** *Chemical Engineering Journal,* 2015, vol. 264, 547
- **GARCIA-SANCHO et al.** *Catalysis Today,* 2011, vol. 167, 84
- **GHOLAMI et al.** *Journal of the Taiwan Institute of Chemical Engineers,* 2013, vol. 44, 117
- **SNYDER ; KIRKLAND.** Introduction to modern liquid Chromatography. John Wiley and Sons, 1979, 248-250